# EUROPÄISCHE PATENTSCHRIFT

(11) **EP 0 648 277 B1**
(45) Veröffentlichungstag und Bekanntmachung des Hinweises auf die Patenterteilung: **17.04.1996**
(21) Anmeldenummer: 93912776.7
(22) Anmeldetag: 25.05.1993
(51) Int. Cl.: C12P 41/00, C12N 9/18, C12N 1/20, C12P 7/22, C12R 1/06

(54) **MIKROBIELLE ESTERASE ZUR ENANTIOSELEKTIVEN SPALTUNG VON 1-ARYLALKYLESTERN**
MICROBIAL ESTERASE FOR THE ENANTIOSELECTIVE CLEAVAGE OF 1-ARYLALKYLESTERS
ESTERASE MICROBIENNE PERMETTANT LA DISSOCIATION ENANTIOSELECTIVE DES ARYLALKYL-1 ESTERS

(30) Priorität: 27.05.1992 DE 4217506
(43) Veröffentlichungstag der Anmeldung: 19.04.1995
(73) Patentinhaber: BOEHRINGER MANNHEIM GMBH, D-68298 Mannheim (DE)
(72) Erfinder: KRELL, Hans-Willi, D-8122 Penzberg (DE); RASOR, Peter, D-8000 München 19 (DE)
(74) Vertreter: Fouquet, Herbert, Dr.
(86) Internationale Anmeldenummer: EP9301302
(87) Internationale Veröffentlichungsnummer: WO9324648

(56) Entgegenhaltungen:
- EP-A- 0 529 085
- AGRICULTURAL AND BIOLOGICAL CHEMISTRY, Bd. 37, Nr. 8, August 1973, TOKYO, JP, Seiten 1923-1928; TAKAYUKI ORITANI ET AL.: "Biochemical resolution of racemic terpenes. IV . Microbial resolution of (+/-) -acyclic alcohols."
- TETRAHEDRON (INCL. TETRAHEDRON REPORTS), Bd. 36, Nr. 1, 1980, OXFORD, GB, Seiten 91-96; KENJI MORI ET AL.: "Synthesis of optically active alkynyl alcohols andalpha-hydrosyesters by microbial asymmetric hydrolysis of the corresponding acetates."
- TETRAHEDRON (INCL. TETRAHEDRON REPORTS), Bd. 47, Nr. 41, 1991, OXFORD, GB, Seiten 8701-8716; HIDENORI DANDA ET AL.: "Preparation of optically active secondary alcohols by combination of enzymic hydrolysis and chemical transformation."

## Beschreibung

Die Erfindung betrifft eine mikrobielle Esterase, welche enantioselektiv 1-Arylalkylester spaltet, ein Verfahren zur Isolierung einer solchen mikrobiellen Esterase, sowie ein Verfahren zur enantioselektiven Spaltung von 1-Arylalkylestern unter Verwendung einer solchen mikrobiellen Esterase.

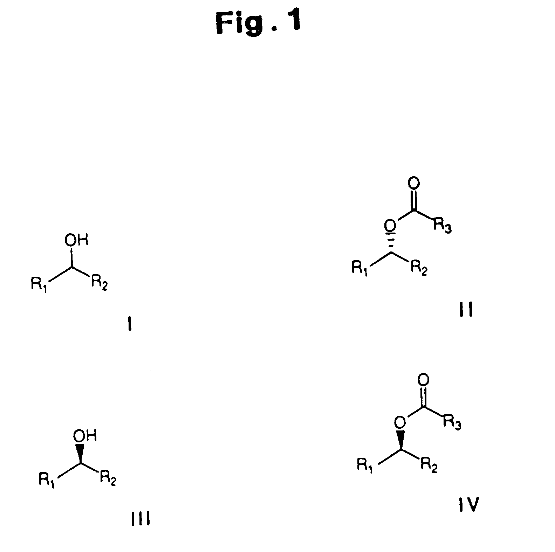

Als Katalysatoren in großtechnischen chemischen Produktionsverfahren finden Enzyme eine weitverbreitete Anwendung. Von besonderer Bedeutung ist hierbei die Enantioselektivität von enzymatisch katalysierten Reaktionen, sowie die Eigenschaft von Enzymen, nicht nur die natürlichen Substrate, sondern auch synthetische Verbindungen entsprechend umzusetzen. Daher können Enzyme auch bei der Synthese chiraler Bausteine von Naturstoffen, Pharmazeutika, Agro- und Feinchemikalien verwendet werden (J. Jones, J. Sih und D. Perlmann, Applications of Biochemical Systems in Organic Chemistry, J. Wiley & Sons, New York 1976, sowie R. Prochter und S. Clark, Enzymes in Organic Synthesis, Pittman, London 1985). Hierbei stellen 1-Arylalkanole (Formel I, s. Fig. 1) wichtige Synthesebausteine für Pharmazeutika dar. Zahlreiche Esterasen und Lipasen, welche 1-Arylalkylester umsetzen und so zu 1-Arylalkanolen führen, sind bekannt und kommerziell erhältlich. Jedoch spalten diese bislang bekannten Esterasen entweder nicht enantioselektiv (Cholesterin-Esterase aus Candida cylindracea und Schweinepankreas, Esterasen aus Kaninchen- und Schweineleber, Lipasen aus Candida cylindracea, Geotrichum candidum, Penicillium roquefortii und Schweinepankreas) oder enantioselektiv nur den 1-Arylalkylester nach Formel II (s. Fig. 1; Lipoproteinlipase und Cholesterin-Esterase aus Pseudomonas spec., Lipase aus Mucor hiemalis, Lipase aus Pseudomonas fluorescens, P. cepacia, P. spec. und Schweinepankreas; s. auch K. Laumen et al., J. Chem. Soc. Chem. Commun. (1988) 598 - 600). Die Enantioselektivität einer Reaktion wird nach C.S. Chen et al. (J. Am. Chem. Soc. 104 (1982), 7294) über den E-Wert charakterisiert. Reaktionen mit einem E-Wert < 20 sind als nicht enantioselektiv anzusehen; Reaktionen mit einem E-Wert von > 50 werden im folgenden als enantioselektiv bezeichnet. Zur Herstellung des 1-Arylalkanols gemäß Formel III (s. Fig. 1) muß bislang auf klassische chemische Verfahren zurückgegriffen werden.

EP-A-0 529 085, welches nach Art. 54(3) EPÜ zu berücksichtigen ist, offenbart die enantioselektive Spaltung von 1-Arylalkylestern wie z.B. der entsprechenden Chlormethylen-substituierten Verbindung 3-Chlor-1-(subst.)-phenyl-1-propylester mit Lipase aus Pseudomonas und anderen Mikroorganismen. Dabei spaltet die Lipase aus Pseudomonas jedoch nur das R-Enantiomer. S-Enantiomere werden nur von Lipasen aus dem Phycomyzeten Rhizopus und Aspergillus niger gespalten

Aus Agricultural and Biological Chemistry 37, 1923-1928 (1973) und Tetrahedron 36, 91-96 (1980) ist die erfindungsgemäße Methode bekannt, wobei jedoch für die Umsetzung Bacillus subtilis und zwar der gesamte Mikroorganismus anstatt der Esterase verwendet wird.

Aufgabe der Erfindung war es daher, eine Esterase zur Verfügung zu stellen, welche enantioselektiv 1-Arylalkylester gemäß Formel IV (s. Fig, 1) hydrolysiert.

Diese Aufgabe wird gelöst durch eine mikrobielle Esterase, welche enantioselektiv (S)-1-Phenylethylacetat spaltet und erhältlich ist aus Arthrobacter spec. (DSM 7034) oder Pseudomonas fluorescens (DSM 7033). Bevorzugt ist die aus Arthrobacter spec. (DSM 7034) erhältliche Esterase.

Der Nachweis der enantioselektiven Spaltungsreaktion erfolgt dabei in bekannter Weise, z. B. über eine HPLC an einem chiralen Säulenmaterial wie z.B. mit Benzoat verestertes Cellulose-beschichtetes Silikagel. Alternativ kann die Enantiomerenreinheit des Produkts auch mit einem Polarimeter bestimmt werden.

Die erfindungsgemäße Esterase weist bei der Bestimmung über eine native Polyacrylamidgel Elektrophorese ein Molekulargewicht von ca. 100.000 D auf und zeigt einen isoelektrischen Punkt von ca. 4,7. Dieses Enzym hydrolysiert substituierte und unsubstituierte 1-Arylalkylester und 1Phenylalkylester, bevorzugt Ester kurzkettiger Carbonsäuren (Kettenlänge 1-7 C-Atome) wie den (S)-1-Phenylethylester der Essigsäure, den (S)-1-Phenylpropylester der Essigsäure oder den (S)-1-(-4-Methylphenyl)-ethylester der Propionsäure). Durch Zusatz von Glycerin kann das Enzym stabilisiert werden.

Zur Isolierung der erfindungsgemäßen Esterase werden die oben genannten Bakterien nach bekannten Verfahren, z. B. durch Behandlung mit Lysozym und Benzonase in hypotonem Puffer, lysiert und der so erhaltene Rohextrakt mit einem löslichen Ionenaustauscher, vorzugsweise Polymin G35 (BASF, Deutschland) behandelt. Die so erhaltene Enzymlösung wird zweimal über eine hydrophobe Chromatographie, vorzugsweise über Phenylsepharose und schließlich über eine Ionenaustauschchromatographie, vorzugsweise an DEAE-Sepharose, weiter aufgereinigt.

Da bislang nur Esterasen beschrieben wurden, welche das in Formel II (s. Fig. 1) gezeigte Enantiomer von 1-Arylalkylestern enantioselektiv spalten, war es überraschend, daß auch eine Esterase gefunden wurde, welche Ester der entgegengesetzten Konfiguration (Formel IV, s. Fig. 1) umsetzt.

Ein weiterer Gegenstand der Erfindung ist ein Verfahren zur Isolierung einer erfindungsgemäßen mikrobiellen Esterase die enantioselektiv (S)-1-Phenylethylacetat spaltet, welches dadurch gekennzeichnet ist, daß man ein Zellysat aus Arthrobacter spec. (DSM 7034), Pseudomonas fluorescens (DSM 7033) oder Bacillus subtilis (DSM 7035) mit einem löslichen Ionenaustauscher behandelt und aus der so behandelten Lösung die Esterase über hydrophobe Chromatographie und Ionenaustausch-Chromatographie isoliert und reinigt.

Als löslicher Ionenaustauscher wird z.B. eine 10 %-ige Polymin G35-Lösung pH 7,0 verwendet. Die hydrophobe Chromatographie und Ionenaustauschchromatographie erfolgt an dem Fachmann bekannten Chromatographiematerialien, wie z.B. Phenylsepharose für die hydrophobe Chromatographie und DEAE-Sepharose für die Ionenaustauschchromatographie. Die Identifizierung der erfindungsgemäßen Esterase im Eluat erfolgt vorzugsweise über die Spaltung von p-Nitrophenylacetat und Messung des freigesetzten p-Nitrophenols.

Ein weiterer Gegenstand der Erfindung sind die Mikroorganismen Arthrobacter spec. DSM 7034, Pseudomonas fluorescens DSM 7033 und Bacillus subtilis DSM 7035.

Die aus diesen Stämmen erhältliche erfindungsgemäße Esterase bewirkt eine enantioselektive Hydrolyse des in Formel IV gezeigten Enantiomers von 1-Arylalkylestern.

Ein weiterer Gegenstand der Erfindung ist ein Verfahren zur enantioselektiven Spaltung eines 1-Arylalkylesters nach Formel IV, wobei R1 ein Aryl- oder substituierter Arylrest und R2 und R3 ein Alkyl- oder substituierter Alkylrest bedeutet, welches dadurch gekennzeichnet ist, daß man ein racemisches Gemisch dieses Esters mit einer erfindungsgemäßen mikrobiellen Esterase behandelt. Hierbei kann sowohl das erfindungsgemäße Enzym als solches, als auch eine Kultur von Mikroorganismen, welche dieses Enzym produzieren, verwendet werden. Die Reaktion erfolgt vorzugsweise in einem wässrigen Puffer, z.B. Kaliumphosphatpuffer, bei einem pH-Wert von 5-10, vorzugsweise 6-9, und einer Temperatur von 5°C - 50°C, vorzugsweise bei 20°C - 40°C. Während der Reaktion wird eine Base, vorzugsweise NaOH, über einen Autotitrator, zugegeben. Wenn ein ausreichender Umsatz, vorzugsweise ein 50 %-iger Umsatz erreicht ist, wird die Reaktion abgebrochen, vorzugsweise durch direkt anschließende Extraktion. Die Reaktion kann aber auch durch Erniedrigung des pH-Wertes, durch Erhitzung oder Zugabe eines Enzyminhibitors wie PMSF (Phenylmethylsulfonylfluorid) abgebrochen werden. Anschließend wird das erhaltene enantiomerenreine 1-Arylalkanol mit einem mit Wasser nicht mischbaren organischen Lösungsmittel extrahiert und gemäß bekannten Verfahren durch Chromatographie oder Destillation von nicht umgesetztem Ester abgetrennt. Die Enantiomerenreinheit wird zweckmäßigerweise über eine HPLC an einem chiralen Säulenmaterial, z.B. mit Benzoat verestertes, cellulosebeschichtetes Silikagel, oder über ein Polarimeter bestimmt.

Ein weiterer Gegenstand der Erfindung ist ein Verfahren zur Herstellung eines 1-Arylalkanols gemäß Formel III, wobei R 1 ein Aryl- oder substituierter Arylrest und R 2 ein Alkyl- oder substituierter Alkylrest bedeuten, welches dadurch gekennzeichnet ist, daß man ein racemisches Gemisch eines 1-Arylalkylesters mit einer erfindungsgemäßen mikrobiellen Esterase in einem wässrigen Puffer bei einem pH-Wert von 5-10, vorzugsweise 6-9 und einer Temperatur von 5°C - 50°C, vorzugsweise von 20°C - 40°C, behandelt und anschließend das erhaltene enantiomerenreine 1-Arylalkanol isoliert.

Da bei der enantioselektiven Spaltung des racemischen 1-Arylalkylesters durch Inkubation mit der erfindungsgemäßen mikrobiellen Esterase nur ein Enantiomer des racemischen Gemischs umgesetzt wird, eignet sich die erfindungsgemäße Esterase auch zur Anreicherung und Isolierung eines enantiomerenreinen 1-Arylalkylesters gemäß Formel II (s. Fig. 1).

Die erfindungsgemäßen Mikroorganismen Arthrobacter spec. DSM 7034, Pseudomonas fluorescens DSM 7033 und Bacillus subtilis DSM 7035 wurden am 03.04.92 bei der Deutschen Sammlung für Mikroorganismen und Zellkulturen GmbH (DSM), Mascheroder Weg 1 b, 3300 Braunschweig, hinterlegt.

Die Erfindung wird durch die nachfolgenden Beispiele zusammen mit der Abbildung näher erläutert:

Figur 1 zeigt die Formeln I - IV von 1-Arylalkylestern bzw. 1-Arylalkanolen, wobei R1 ein Aryl- oder substituierter Arylrest und R 2 und R 3 ein Alkyl- oder substituierter Alkylrest bedeuten.

### Beispiel 1:

Isolierung einer Esterase aus Arthrobacter spec., welche enantioselektiv (S)-1-Phenylethylacetat spaltet

Die Kultivierung von Arthrobacter spec. erfolgt in einem Medium, welches 15,6 g/l Spezialpepton (E. Merck, Deutschland), 2,8 g/l Hefeextrakt (E. Merck), 5,6 g/l Natriumchlorid und 1 g/l Glucose enthält und einen pH-Wert von 7,0 aufweist, für 16 Stunden bei 28°C. Zum Zellaufschluß wird das abzentrifugierte Bakterienpellet zu einer 20 %-igen (w/v) Suspension in 50 mmol/l Kaliumphosphatpuffer pH 7,5 aufgenommen und mit 10 mg/ml Lysozym, sowie 300 U/ml Benzonase (E. Merck, Deutschland) unter Rühren für 2 Stunden bei 25°C inkubiert.

Zu 1 ml des erhaltenen 20 %-igen Rohextrakts werden 80 µl einer 10 % Polymin G 35 Lösung, pH 7,0 (BASF, Deutschland) pipettiert, 5 Min. unter Rühren bei 25°C inkubiert und anschließend bei 18.000 g für 20 Minuten abzentrifugiert.

Die nach Polyminbehandlung erhaltene klare Enyzmlösung wird auf 10 mmol/l Kaliumphosphatpuffer pH 7,0, 10 % Ammoniumsulfat und 0,1 mmol/l Dithioerythritol (DTE) eingestellt. Diese Proteinprobe wird dann auf eine mit 10 mmol/l Kaliumphosphatpuffer pH 7,0, 10 % Ammoniumsulfat und 0,1 mmol/l DTE äquilibrierte Phenylsepharose fast flow Säule (2,0 x 6 cm, Pharmacia, Schweden ; Sepharose ist ein Warenzeichen) aufgetragen. Nach Auswaschen von ungebundenen Substanzen mit dem oben angegebenen Äquilibrierungspuffer wird gebundene Esterase mit 10 mmol/l Kaliumphosphatpuffer pH 7,0/0,1 mmol/l DTE eluiert. Die aktiven Fraktionen werden über die Spaltung von p-Nitrophenylacetat (gemäß Beispiel 2) identifiziert, vereinigt und gegen 10 mmol/l Kaliumphosphatpuffer pH 7,0/0,1 mmol/l DTE, gesättigt mit Polyethylenglykol (PEG 6000), dialysiert und dabei konzentriert. Das erhaltene Konzentrat wird erneut auf eine Phenylsepharosesäule gegeben, die jedoch in diesem Fall mit 100 mmol/l Kaliumphosphatpuffer pH 7,0/0,1 mmol/l DTE äquilibriert wurde. Die Elution erfolgt mit einem Gradienten von 100 mmol/l auf 10 mmol/l Kaliumphosphatpuffer bei einer Flußrate von 5 ml/min und einer Fraktionsgröße von je 10 ml. Die erhaltenen aktiven Fraktionen werden erneut vereinigt und gegen 20 mmol/l Kaliumphosphatpuffer pH 7,5/0,1 mmol/l DTE, gesättigt mit PEG 6000, dialysiert und dabei konzentriert.

Das erhaltene Konzentrat wird schließlich auf eine DEAE Sepharose fast flow Säule (5 x 1,2 cm), die mit 20 mmol/l Kaliumsphosphatpuffer pH 7,5, 0,1 mmol/l DTE äquilibriert wurde, aufgetragen und mit dem 3-fachen Säulenvolumen nachgewaschen.
Die Elution erfolgt mit einem Gradienten von 0-0,3 mol/l Natriumchlorid in 20 mmol/l Kaliumphosphat pH 7,5/0,1 mmol/l DTE bei einer Durchflußrate von 2,5 ml/min. Die Esterase eluiert bei einem Salzgehalt von ca. 0,3 mol/l Natriumchlorid. Die aktiven Fraktionen werden vereinigt und gegen 10 mmol/l Kaliumphosphatpuffer pH 7,0/0,1 mmol/l DTE, gesättigt mit PEG 6000, dialysiert und dabei konzentriert. Dieser Lösung wird zur Stabilisierung Glycerin bis zu einer Endkonzentration von 60 Vol.- % zugegeben.

### Beispiel 2:

### Bestimmung der Esteraseaktivität

In eine Küvette mit 1 cm Schichtdicke werden 1800 µl 0,2 mol/l Kaliumphosphatpuffer pH 6,5, 100 µl p-Nitrophenylacetat (20 mmol/l in Ethanol) und 100 µl der zu testenden Enzymlösung gegeben. Die Messung erfolgt bei 30°C und 410 nm für 1 Minute. Der Extinktionskoeffizient beträgt 15 x 10⁶ cm/mol. Ein Unit ist definiert als die Menge, die 1 µmol p-Nitrophenylacetat in einer Minute unter diesen Testbedingungen hydrolysiert.

### Beispiel 3:

### Enzymatische Racematspaltung von 1-Phenylethylacetat

Zur präparativen Spaltung wird racemisches 1-Phenylethylacetat (50 mmol/l) in 10 mmol/l Phosphatpuffer pH 7,0 mit der gemäß Beispiel 1 isolierten erfindungsgemäßen Esterase (1 Unit Esterase je 1 mmol Ester) bei 30°C inkubiert. Der pH-Wert wird während der Reaktion mittels einer automatischen Bürette, welche 0,1 mol/l NaOH zutitriert, konstant gehalten. Der Reaktionsverlauf wird an Hand des Natronlaugeverbrauchs verfolgt. Der Enantiomerenüberschuß des Produkts 1-Phenylethanol, sowie des Substrats 1-Phenylethylacetat, wird durch HPLC an einer chiralen Säule (Chiralcel OJ, Firma Daicel) bestimmt. Ist ein 50 %iger Umsatz erreicht, beträgt der Enantiomerenüberschuß des zurückbehaltenen Substrats (R)-1-Phenylethylacetat 96 % und des Produkts (S)-1-Phenylethanol 94 % (Enantioselektivität E > 100). Die Reaktion wird abgebrochen, indem mehrfach mit Essigsäureethylester extrahiert wird. Die organischen Phasen werden vereinigt und durch Verdampfen des Lösungsmittels eingeengt. Der erhaltene (S)-1-Phenylethanol wird von nicht umgesetztem (R)-1-Phenylethylacetat durch Chromatographie an Silikagel abgetrennt.

## Patentansprüche

1. Mikrobielle Esterase, dadurch gekennzeichnet, daß sie enantioselektiv (S)-1-Phenylethylacetat spaltet und erhältlich ist aus Arthrobacter spec. DSM 7034) oder Pseudomonas fluorescens (DSM 7033).

2. Mikrobielle Esterase gemäß Anspruch 1, dadurch gekennzeichnet, daß sie aus Arthrobacter spec. (DSM 7034) erhältlich ist.

3. Verfahren zur Isolierung einer mikrobiellen Esterase, die enantioselektiv (S)-1-Phenylethylacetat spaltet, dadurch gekennzeichnet, daß man ein Zellysat aus Arthrobacter spec. (DSM 7034) oder Pseudomonas fluroescens (DSM 7033) mit einem löslichen Ionenaustauscher behandelt und aus der so behandelten Lösung die Esterase über hydrophobe Chromatographie und Ionenaustauschchromatographie isoliert und reinigt.

4. Arthrobacter spec. DSM 7034.

5. Pseudomonas fluorescens DSM 7033.

6. Verfahren zur enantioselektiven Spaltung eines 1-Arylalkylesters nach Formel IV, 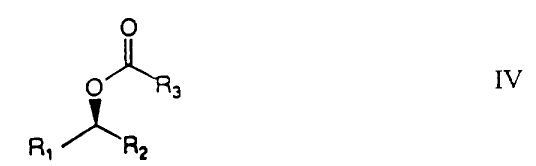 wobei R1 ein Aryl- oder substituierter Arylrest und R2 und R3 ein Alkyl- oder substituierter Alkylrest bedeuten, dadurch gekennzeichnet, daß man ein racemisches Gemisch dieses Esters mit einer mikrobiellen Esterase gemäß Anspruch 1 in einem wässrigen Puffer bei einem pH-Wert von 5-10 und einer Temperatur von 5°C - 50°C behandelt.

7. Verfahren nach Anspruch 6, dadurch gekennzeichnet, daß man den Ester mit der mikrobiellen Esterase bei einem pH-Wert von 6-9 und einer Temperatur von 20°C - 40°C behandelt.

8. Verfahren zur Herstellung eines 1-Arylalkanols nach Formel III, 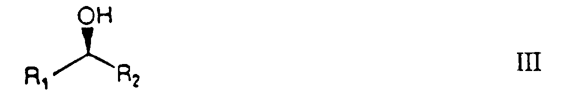 wobei R1 ein Aryl- oder substituierter Arylrest und R2 ein Alkyl oder substituierter Alkylrest bedeuten, dadurch gekennzeichnet, daß man ein racemisches Gemisch eines 1-Arylalkylesters mit einer mikrobiellen Esterase gemäß Anspruch 1 in einem wässrigen Puffer bei einem pH-Wert von 5-10 und einer Temperatur von 5°C - 50°C behandelt und anschließend das erhaltene 1-Arylalkanol isoliert.

9. Verfahren nach Anspruch 8, dadurch gekennzeichnet, daß man den 1-Arylalkylester mit der mikrobiellen Esterase bei einem pH-Wert von 6-9 und einer Temperatur von 20°C - 40°C behandelt.

## Claims

1. Microbial esterase, wherein it enantioselectively cleaves (S)-1-phenylethyl acetate and is obtainable from Arthrobacter spec. (DSM 7034) or Pseudomonsas fluorescens (DSM 7033).

2. Microbial esterase as claimed in claim 1, wherein is is obtainable from Arthrobacter spec. (DSM 7034).

3. Process for the isolation of a microbial esterase which enantioselectively cleaves (S)-1-phenylethyl acetate, wherein a cell lysate from Arthrobacter spec. (DSM 7034) or Pseudomonas fluorescens (DSM 7033) is treated with a soluble ion exchanger and the esterase is isolated and purified from the solution treated in this manner by hydrophobic chromatography and ion-exchange chromatography.

4. Arthrobacter spec. DSM 7034.

5. Pseudomonas fluorescens DSM 7033.

6. Process for the enantioselective cleavage of a 1-arylalkyl ester of formula IV, 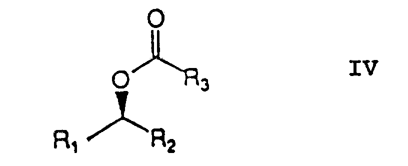 in which R1 denotes an aryl or substituted aryl residue and R2 and R3 denote an alkyl or substituted alkyl residue, wherein a racemic mixture of this ester is treated with a microbial esterase as claimed in claim 1 in an aqueous buffer at a pH value of 5-10 and at a temperature of 5°C - 50°C.

7. Process as claimed in claim 6, wherein the ester is treated with the microbial esterase at a pH value of 6-9 and at a temperature of 20°C - 40°C.

8. Process for the production of a 1-arylalkanol according to formula III, 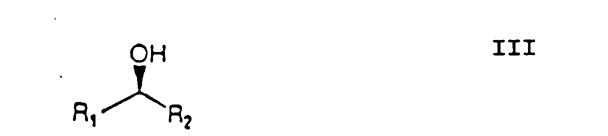 in which R1 denotes an aryl or substituted aryl residue and R2 denotes an alkyl or substituted alkyl residue, wherein a racemic mixture of a 1-arylalkyl ester is treated with a microbial esterase as claimed in claim 1 in an aqueous buffer at a pH value of 5-10 and at a temperature of 5°C - 50°C and subsequently the 1-arylalkanol obtained is isolated.

9. Process as claimed in claim 8, wherein the 1-arylalkyl ester is treated with the microbial esterase at a pH value of 6-9 and at a temperature of 20°C - 40°C.

## Revendications

1. Estérase microbienne, caractérisée par le fait qu'elle coupe de façon énantiosélective le (S)-1-phényléthylacétate et qu'elle peut être obtenue à partir d'une espèce d'Arthrobacter (DSM 7034) ou de Pseudomonas fluorescens (DSM 7033).

2. Estérase microbienne selon la revendication 1, caractérisée par le fait qu'elle peut être obtenue à partir d'espèces d'Arthrobacter (spec. DSM 7034).

3. Procédé d'isolement d'une estérase microbienne qui coupe de façon énantiosélective le (S)-1-phényléthylacétate, caractérisé par le fait que l'on traite un lysat cellulaire d'une espèce d'Arthrobacter (DSM 7034) ou de Pseudomonas fluorescens (DSM 7033) avec un échangeur d'ions soluble, et qu'à partir de la solution ainsi traitée, on isole et purifie l'estérase par chromatographie hydrophobe et chromatographie par échange d'ions.

4. Arthrobacter spec. DSM 7034.

5. Pseudomonas fluorescens DSM 7033.

6. Procédé de clivage énantiosélectif d'un 1-arylalkylester de formule IV 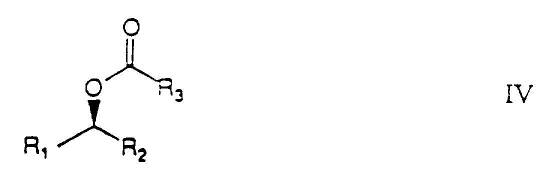 dans laquelle R1 est un reste aryle ou aryle substitué et R2 et R3 représentent un reste alkyle ou alkyle substitué, caractérisé par le fait que l'on traite un mélange racémique de cet ester avec une estérase microbienne selon la revendication 1, dans un tampon aqueux à un pH de 5-10 et une température de 5°C-50°C.

7. Procédé selon la revendication 6, caractérisé par le fait que l'on traite l'ester avec l'estérase microbienne à un pH de 6-9 et une température de 20°C-40°C.

8. Procédé de préparation d'un 1-arylalcanol de formule III 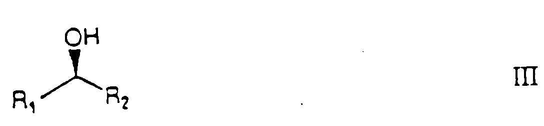 dans laquelle R1 est un reste aryle ou aryle substitué et R2 représente un reste alkyle ou alkyle substitué, caractérisé par le fait que l'on traite un mélange racémique d'un 1-arylalkylester avec une estérase microbienne selon la revendication 1, dans un tampon aqueux à un pH de 5-10 et une température de 5°C-50°C, et qu'ensuite, on isole le 1-arylalcanol obtenu.

9. Procédé selon la revendication 8, caractérisé par le fait que l'on traite le 1-arylalkylester avec l'estérase microbienne à un pH de 6-9 et une température de 20°C-40°C.
